# EUROPEAN PATENT APPLICATION

(11) **EP 4 692 073 A1**
(43) Date of publication of application: **11.02.2026**
(21) Application number: 24777859.0
(22) Date of filing: 21.03.2024
(51) Int. Cl.: C07D 401/14, C07D 207/08, A61K 31/4025, A61P 3/06

(54) **CYCLIC AMINE DERIVATIVE, AND COMPOSITION AND USE THEREOF**

(30) Priority: 24.03.2023 CN 202310301299
(71) Applicant: Shanghai Jingxin Biomedical Co., Ltd., Shanghai 201210 (CN); Zhejiang Jingxin Pharmaceutical Co., Ltd., Shaoxing, Zhejiang 312500 (CN)
(72) Inventor: NI, Shuaijian, Shanghai 201210 (CN); SONG, Ying, Shanghai 201210 (CN); TANG, Jiaqi, Shanghai 201210 (CN)
(74) Representative: Gille Hrabal Partnerschaftsgesellschaft mbB Patentanwälte
(86) International application number: PCT/CN2024/082950
(87) International publication number: WO 2024/199070

(57) **Abstract**

The present invention relates to a cyclic amine derivative or a pharmaceutically acceptable salt thereof, a pharmaceutical composition comprising the cyclic amine derivative or the pharmaceutically acceptable salt thereof, and the use of the cyclic amine derivative or the pharmaceutically acceptable salt thereof or the composition thereof. The cyclic amine derivative of the present invention has a relatively strong capacity of binding to apo(a), can effectively reduce the level of Lp(a), and has low toxic side effects.

## Description

The present application claims the priority of the patent application, which was filed with the China Patent Office on March 24, 2023, with application number 202310301299.3 and application name "A cyclic amine derivative, and composition and use thereof", the entire contents of which are incorporated herein by reference.

### TECHNICAL FIELD

The present invention relates to a cyclic amine derivative or a pharmaceutically acceptable salt thereof, a pharmaceutical composition comprising the cyclic amine derivative or the pharmaceutically acceptable salt thereof, and the use of the cyclic amine derivative or the pharmaceutically acceptable salt thereof or the composition thereof.

### BACKGROUND

Hyperlipidemia is a major risk factor for atherosclerosis and is closely associated with cardiovascular diseases, posing a serious threat to human health. Within the human body, blood lipids must bine with apolipoproteins to form lipoproteins in order to be soluble in the blood and transported to tissues for metabolism. Lipoproteins include chylomicrons (CM), very low-density lipoproteins (VLDL), intermediate density lipoproteins (IDL), low-density lipoproteins (LDL), high-density lipoproteins (HDL) and lipoproteins (Lp(a)).

Lp(a) is readily deposited on the vascular wall and can lead to the formation of atherosclerotic lesions through multiple mechanisms, its atherogenic potential is no less than that of LDC-C, In addition, Lp(a) also exhibits prothrombotic properties. After being synthesized in the liver,Lp(a) is secreted into the blood circulation, and predominantly deposited in vascular tissues and aortic valve leaflets.

The lipid composition of Lp(a) is similar to that of LDL, the difference is that Lp(a) contains a unique apolipoprotein apo(a), which is combined with apoB100 by disulfide bond, apo(a) is a highly glycosylated hydrophilic protein with structural polymorphism, accounting for 25% - 40% of the total protein content of Lp(a), which is the key to Lp(a)'s specific role in causing atherosclerotic cardiovascular disease (ASCVD).

Patent WO2020/247429A1 discloses a series of pharmaceutically acceptable compounds for reducing plasma Lp(a) levels, which can be used in the preparation of drugs for cardiovascular diseases. Although certain progress has been made in the development of drugs for treating cardiovascular diseases in this field, there is still necessary to further develop cardiovascular drugs that meet clinical needs.

### SUMMARY OF THE INVENTION

The present invention provides a cyclic amine derivative which has a strong binding affinity for apo(a), effectively lowers Lp(a) levels, and has low toxicity and side effects.

Specifically, the present invention provides a compound of formula I or a pharmaceutically acceptable salt thereof: 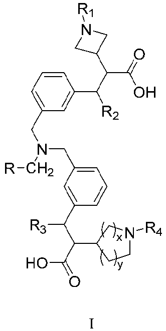 wherein,
R is selected from aryl or heteroaryl, which may be further substituted;
R₁, R₂, R₃ and R₄ are each independently selected from H and alkyl, the alkyl may be further substituted;
x is 1 or 2, preferably 1;
y is 0 or 1;
In some embodiments, R is selected from C6-C20 aryl, preferably C6-C14 aryl, more preferably C6-C12 aryl, which may be further substituted;
In some embodiments, R₁, R₂, R₃ and R₄ are each independently selected from H and C1-6 alkyl;
In some embodiments, R₁, R₂, R₃ and R₄ are each independently selected from H and methyl, preferably H;
In some embodiments, R is selected from phenyl, benzo[d][1,3]dioxole, tetrahydroquinoline, tetrahydroisoquinoline, pyridine, quinoline or isoquinoline, which may be further substituted;
In some embodiments, R is selected from the following groups: 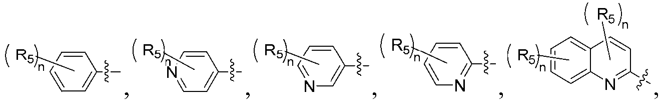 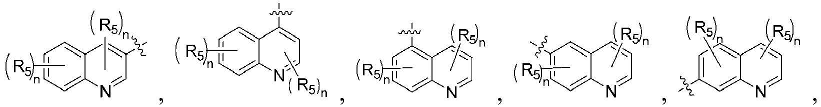 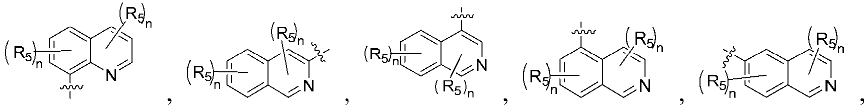 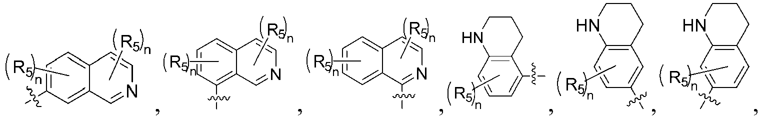 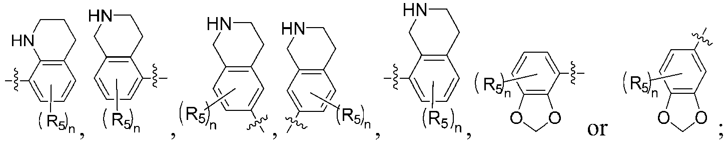
Wherein, n is 0, 1, 2 or 3;
R₅ is each independently selected from hydrogen, alkyl, haloalkyl, alkoxy, haloalkoxy, alkylamino, alkylcarbonylamino, halogen, hydroxy, nitro, cyano, cycloalkyl, aryl or heteroaryl; preferably alkyl, haloalkyl, alkoxy, haloalkoxy, alkylamino, alkylcarbonylamino or halogen;
the R₅ may be located at one, two, or three positions selected from the 1st, 2nd, 3rd, 4th, 5th, 6th, 7th and 8th positions of the aforementioned aryl or heteroaryl.

In some embodiments, R₅ is each independently selected from C1-6 alkyl, C3-6 cycloalkyl, C1-6 haloalkyl, C1-6 alkoxy, C1-6 haloalkoxy, C1-6 alkylcarbonylamino, halogen or 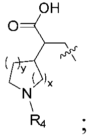 wherein x, y, and R₄ are as defined above.

In some embodiments, R₅ is each independently selected from trifluoromethyl, 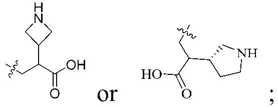 wherein, preferably 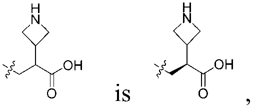 preferably

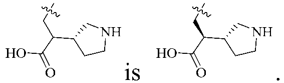

In some embodiments, R is selected from the following groups:

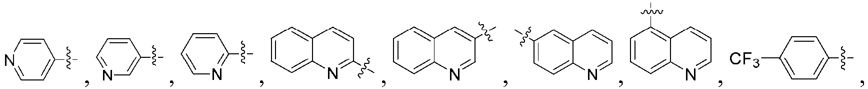

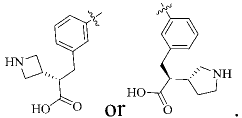

In the present invention, the carbon in 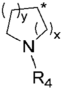 may be in the R or S configuration, preferably in the R configuration;
In the present invention, the carbon atom which is connected to the cyclic amine and the carboxyl group may be in the R or S configuration, preferably in the S configuration.

Specifically, the present invention also provides a compound of formula II or formula III or a pharmaceutically acceptable salt thereof: 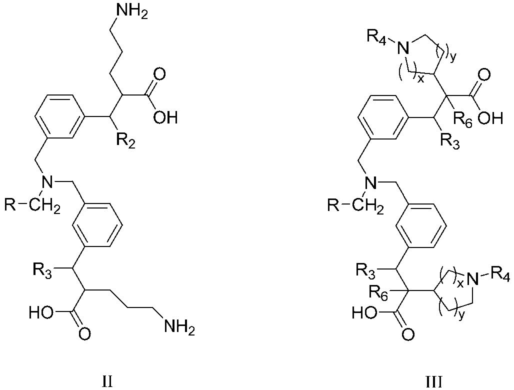 wherein,
R, R₂, R₃, R₄, x, and y are as defined above;
R₆ is each independently selected from C1-6 alkyl; preferably methyl;
In the present invention, , the carbon in 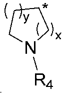 may be in the R or S configuration, preferably in the R configuration;
In the present invention, the carbon atom which is connected to the cyclic amine and the carboxyl group may be in the R or S configuration, preferably in the S configuration.

Specifically, the present invention also provides a compound of formula IV or a pharmaceutically acceptable salt thereof: 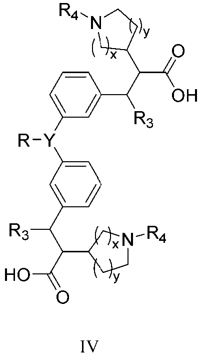 wherein,
Y is a trivalent group, the trivalent group is selected from 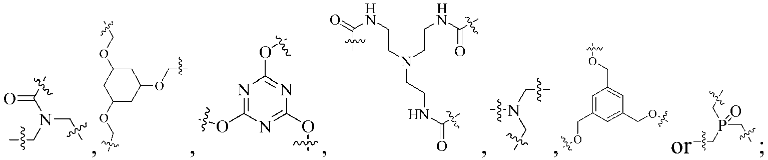
R, R₃, R₄, x, and y are as defined above;
In some embodiments, the compound of formula IV or the pharmaceutically acceptable salt thereof may be a compound of formula IV-1, formula IV-2, formula IV-3, or formula IV-4, or a pharmaceutically acceptable salt thereof: 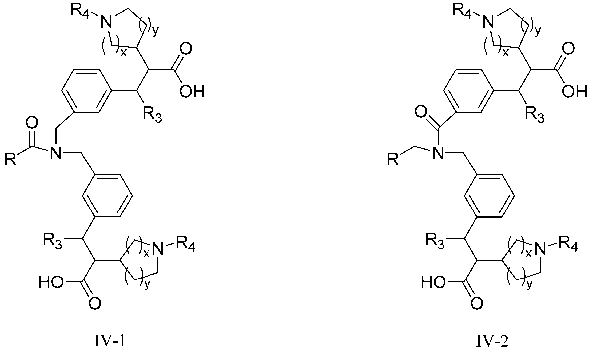 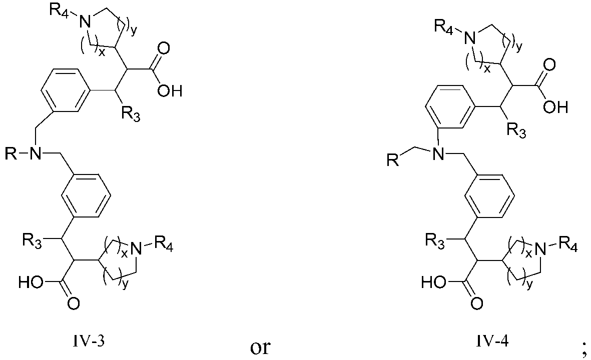
R, R₃, R₄, x, and y are as defined above;
In the present invention, the carbon in 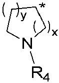 may be in the R or S configuration, preferably in the R configuration;
In the present invention, the carbon atom connected to the cyclic amine and the carboxyl group may be in the R or S configuration, preferably in the S configuration.

In some embodiments, the compound of the present invention is selected from:

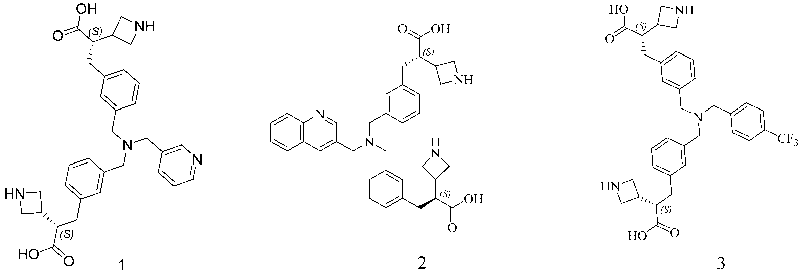

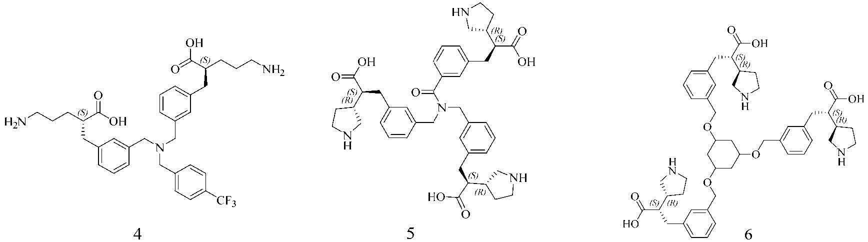

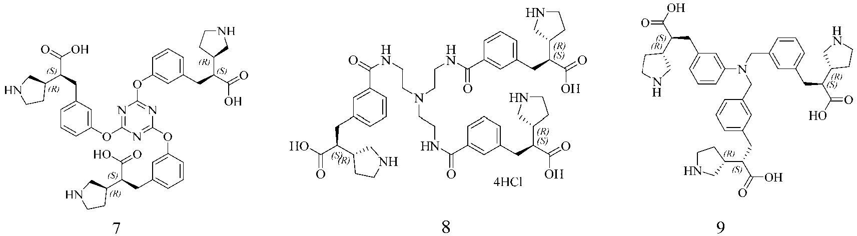

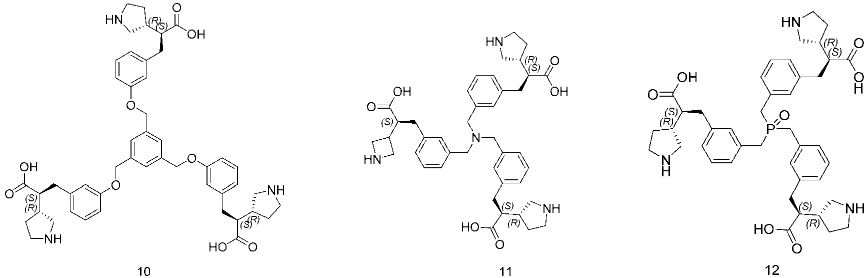

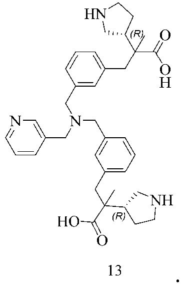

The present invention also relates to a pharmaceutical composition, comprising the compound of the present invention or a pharmaceutically acceptable salt thereof.

In some embodiments, the pharmaceutical composition optionally comprises the pharmaceutically acceptable excipient.

The present invention also relates to a method for treating cardiovascular diseases, comprising administering an effective amount of the compound of the present invention or a pharmaceutically acceptable salt thereof to a patient.

The present invention also relates to a method of treating a patient in need of treatment for elevated Lp(a) plasma levels, comprising administering an effective amount of the compound of the present invention or a pharmaceutically acceptable salt thereof to patients.

The present invention also relates to a method for inhibiting the assembly of LDL particles with apo(a), comprising binding the compound of the present invention or a pharmaceutically acceptable salt thereof to apo(a).

The present invention also relates to the compound of the present invention, or a pharmaceutically acceptable salt thereof, or the aforementioned composition, for use in the preparation of an apo(a) binder.

In some embodiments, the compound of the present invention, or a pharmaceutically acceptable salt thereof, or the aforementioned composition, is used to prepare an inhibitor of the assembly of LDL particles with apo(a).

In some embodiments, the compound of the present invention, or a pharmaceutically acceptable salt thereof, or the aforementioned composition, is used to prepare an agent for reducing Lp(a) levels.

In some embodiments, the compound of the present invention, or a pharmaceutically acceptable salt thereof, or the aforementioned composition, is used to prepare a drug for treating cardiovascular.

### DETAILED DESCRIPTION OF THE INVENTION

Before the present invention is further described, it should be understood that the invention is not limited to the embodiments described, as such may, of course, vary. It should also be understood that the terminology used herein is for the purpose of describing the embodiments only, and is not intended to be limiting, since the scope of the present invention will be limited only by the appended claims.

Unless otherwise indicated, all technical and scientific terms used herein have the same meaning which is commonly understood by those of ordinary skill in the art to which this invention belongs. All patents, applications, published applications and other publications referred to herein are incorporated by reference in their entireties. If the definition in this section is contrary to or inconsistent with the definition in the patents, applications, or other publications incorporated herein by reference, the definition in this section will prevail over the definition incorporated herein by reference.

In the present invention, "alkyl" refers to a saturated aliphatic hydrocarbon group, which may be a C1-20 alkyl group, preferably a C1-8 alkyl group, more preferably a C1-6 alkyl group, and most preferably a C1-3 alkyl group. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, and various branched isomers thereof, etc.

In the present invention, "haloalkyl" refers to an alkyl group substituted with one or more (preferably 1, 2, 3, 4, 5 or 6) halogen atoms, wherein the alkyl group is as defined above, which may be a C1-20 haloalkyl group, preferably a C1-8 haloalkyl group, more preferably a C1-6 haloalkyl group, and most preferably a C1-3 haloalkyl group. Non-limiting examples include trifluoromethyl, monofluoromethyl, difluoromethyl, trichloromethyl, pentafluoroethyl, etc;
In the present invention, "alkoxy" refers to alkyl-O-, wherein alkyl is as defined above;
In the present invention, "haloalkoxy" refers to haloalkyl-O-, wherein haloalkyl is as defined above;
In the present invention, "alkylamino" refers to alkyl-NH-, wherein alkyl is as defined above;
In the present invention, "alkylcarbonylamino" refers to alkyl-C(O)-NH-, wherein the alkyl group is as defined above;
In the present invention, "halogen" refers to fluorine, chlorine, bromine, or iodine;
In the present invention, "cycloalkyl" refers to a saturated or partially unsaturated monocyclic or polycyclic hydrocarbon substituent, the ring of cycloalkyl contains 3 to 20 carbon atoms, preferably 3 to 12 carbon atoms, and more preferably 3 to 6 carbon atoms. Non-limiting examples of cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl,etc.

In the present invention, the alkyl, haloalkyl, alkoxy, haloalkoxy, alkylamino, alkylcarbonylamino, cycloalkyl, etc, may be substituted or unsubstituted, and when it is substituted, the substituent may be substituted at any accessible point of attachment, the substituent is preferably one or more of the following groups, independently selected from the group consisting of alkyl, haloalkyl, alkenyl, alkynyl, alkoxy, haloalkoxy, alkylthio, alkylamino, alkylcarbonylamino, halogen, mercapto, hydroxyl, nitro, cyano, cycloalkyl, heterocycloalkyl, aryl, heteroaryl, cycloalkyloxy, heterocycloalkyloxy, cycloalkylthio, heterocycloalkylthio, oxo, carboxy, or carboxylate.

In the present invention, "aryl" refers to an optionally substituted monocyclic, biaryl or fused bicyclic or polycyclic ring system, having the well-known characteristics of aromaticity, wherein at least one ring contains a completely conjugated π-electron system. Typically, aryl groups contain 6 to 20 carbon atoms ("C6-C20 aryl") as ring members, preferably 6 to 14 carbon atoms ("C6-C14 aryl"), or more preferably 6 to 12 carbon atoms ("C6-C12 aryl"). Fused aryl groups may include an aryl ring (e.g., a phenyl ring) fused to another aryl ring, or fused to a saturated or partially unsaturated carbocyclic or heterocyclic ring. The point of attachment to the base molecule on such fused aryl ring systems may be a C atom of the aromatic portion or a C or N atom of the non-aromatic portion of the ring system. Examples, without limitation, of aryl groups include phenyl, biphenyl, naphthyl, anthracenyl, phenanthrenyl, indanyl, indenyl, Benzo[d][1,3]dioxole, and tetrahydronaphthyl.

In the present invention, "heteroaryl" refers to monocyclic, heterobiaryl or fused bicyclic or polycyclic ring systems having the well-known characteristics of aromaticity that contain the specified number of ring atoms and include at least one heteroatom selected from N, O and S as a ring member in an aromatic ring. The inclusion of a heteroatom permits aromaticity in 5-rmembered rings as well as 6-membered rings. Typically, heteroaryl groups contain 5 to 20 ring atoms ("5-20 membered heteroaryl"), preferably 5 to 14 ring atoms ("5-14 membered heteroaryl"), and more preferably 5 to 12 ring atoms ("5-12 membered heteroaryl"). Heteroaryl rings are attached to the base molecule by a ring atom of the heteroaromatic ring, such that aromaticity is maintained. Thus, 6-membered heteroaryl rings may be attached to the base molecule via a ring C atom, while 5-membered heteroaryl rings may be attached to the base molecule by a ring C or N atom. Examples of unsubstituted heteroaryl groups often include, but are not limited to, pyrrole, furan, thiophene, pyrazole, imidazole, isoxazole, oxazole, isothiazole, thiazole, triazole, oxadiazole, thiadiazole, tetrazole, pyridine, pyridazine, pyrimidine, pyrazine, benzofuran, benzothiophene, indole, benzimidazole, indazole, quinoline, isoquinoline, purine, triazine, naphthyridine and carbazole.

In the present invention, "aryl" or "heteroaryl" may be optionally substituted or unsubstituted, when substituted, the substituent is preferably one or more of the following groups, independently selected from alkyl, haloalkyl, alkenyl, alkynyl, alkoxy, haloalkoxy, alkylthio, alkylamino, alkylcarbonylamino, halogen, mercapto, hydroxyl, nitro, cyano, cycloalkyl, heterocycloalkyl, aryl, heteroaryl, cycloalkyloxy, heterocycloalkyloxy, cycloalkylthio, heterocycloalkylthio, carboxy, or carboxylate.

In the present invention, "elevated Lp(a) plasma levels" means a plasma level of Lp(a) that is equal to or above about 50 mg/dL.

In the present invention, any isotopically-labeled derivative of the compound or the pharmaceutically acceptable salt thereof described herein is encompassed by the present disclosure. Atoms that can be isotopically labeled include, but are not limited to, hydrogen, carbon, nitrogen, oxygen, phosphorus, fluorine, chlorine, iodine, etc. They may be separately replaced by the isotopes ²H(D), ³H, ¹¹C, ¹³C, ¹⁴C, ¹⁵N, ¹⁸F, ³¹P, ³²P, ³⁵S, ³⁶Cl and ¹²⁵I, etc. Unless otherwise indicated, when a position is specifically designated as deuterium (D), that position shall be understood to be deuterium having an abundance that is at least 3000 times greater than the natural abundance of deuterium (which is 0.015%) (i.e., incorporating at least 45% deuterium).

In the present invention, "more" means at least two , for example, 2, 3, 4, 5 or 6, etc.

In the present invention, "optional" or "optionally" means that the event or circumstance subsequently described may occur, but does not necessarily occur, and this description includes instances where the event or circumstance occurs or does not occur. For example, "Heterocyclic group that is optionally substituted with an alkyl" means that the alkyl may present, but does not necessarily present, and this description includes the instance where the heterocyclic group is substituted with an alkyl and the instance where the heterocyclic group is not substituted with an alkyl.

In the present invention, "each independently" or "independently" means that substituents with the same selection range can be the same or different groups at each occurrence, and the group selection of the substituent at each occurrenc is not affected by the selection of the substituent (or substituents with the same selection range) at other positions.

In the present invention, "pharmaceutically acceptable salt" refers to the salt of the compound of the present invention, which is safe and effective when used in mammals and has the desired biological activity.

In the present invention, the following abbreviations/terms are used:
Apo(a): apolipoprotein(a)
Lp(a): lipoprotein(a)
TEA: triethylamine
THF: tetrahydrofuran
LiHMDS: lithium hexamethyldisilazide
PE: petroleum ether
EtOAc: ethyl acetate
MTBE: methyl tert-butyl ether
2-MeTHF: 2-methyltetrahydrofuran
DMAP: 4-dimethylaminopyridine
Boc₂O: di-tert-butyl carbonate
HATU: 2-(7-azabenzotriazole)-N,N,N',N'-tetramethyluronium hexafluorophosphate
DIEA: N,N-diisopropylethylamine
NMP: N-methylpyrrolidone
SPR: surface plasmon resonance
EDC: 1-ethyl-3-(3-dimethylaminopropyl)carbodiimide hydrochloride
NHS: N-hydroxysuccinimide
NaAc: sodium acetate
RU: response unit
BLI: biolayer Interferometry
PBS: phosphate buffered saline
BSA: bovine serum albumin
HEC: hydroxyethylcellulose
EDTAK2: ethylenediaminetetraacetic acid dipotassium salt

### EXAMPLES

### Example 1

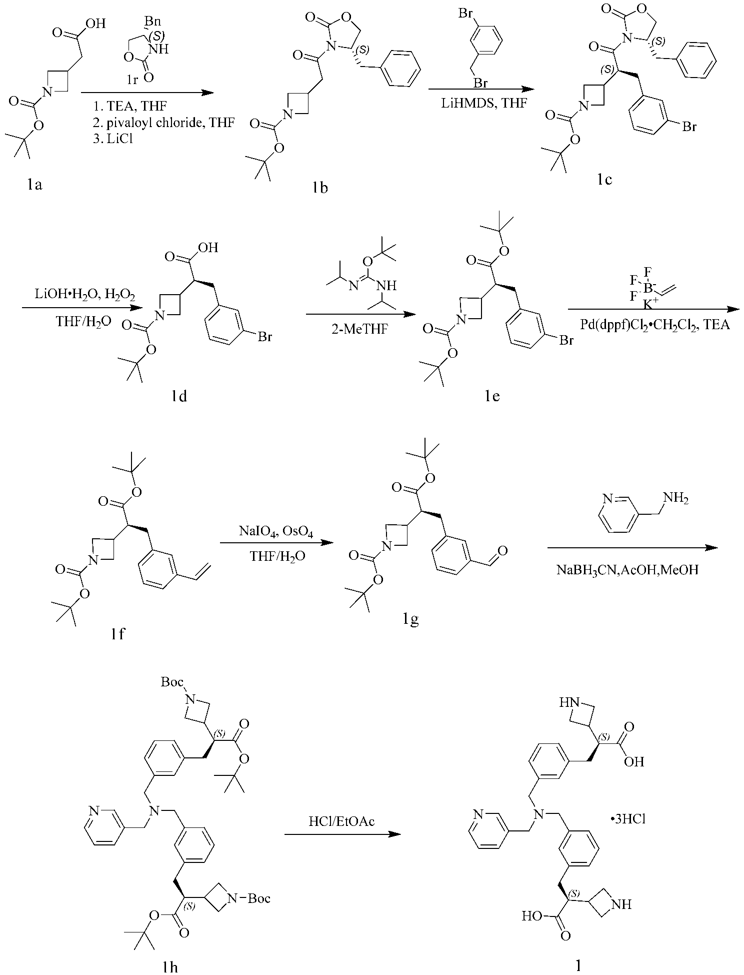

### Step 1:

Compound 1a (53.8 g, 249.94 mmol) was dissolved in THF (300 mL), TEA (63.23 g, 624.855 mmol) was added under ice bath conditions, pivaloyl chloride (37.67 g, 312.43 mmol) was added after stirring for 5 minutes under ice bath conditions, and stirred for 15 minutes under ice bath conditions. LiCl (13.24 g, 312.427 mmol) and (4S)-4-benzyl-1,3-oxazole-2-one (44.29 g, 249.94 mmol) were dissolved in THF (400 mL) in advance and stirred until dissolved. The LiCl/(4S)-4-benzyl-1,3-oxazole-2-one solution was added to the compound 1a reaction solution, and the mixture was stirred at room temperature for 24 hours. The white solid was formed in the reaction solution. 1M HCl was added until the solid disappeared, the organic phase was separated, washed once with 1M NaOH aqueous solution (350 mL), and then washed once with saturated brine, dried over anhydrous sodium sulfate, filtered, and concentrated to give a yellow oily product. MeOH:H₂O (1:2) (600 mL) was added and slurried overnight, the slurry was filtered, the filter cake was dried to obtain white solid 1b (68 g, yield 72.66%).
MS m/z(ESI):319.2[M-56] ⁺.

### Step 2:

Compound 1b (11 g, 29.38 mmol) was dissolved in THF (100 mL), and LiHMDS (1 M in hexane, 41.13 mL) was added at -10 °C. After maintaining the temperature for 15 minutes, the solution(25mL) of m-bromobenzyl bromide (8.08 g, 32.32 mmol) in THF was added, and slowly heated to room temperature and reacted overnight. Quenched with saturated ammonium chloride solution (20 mL), water was added, and the mixture was extracted twice with EtOAc, washed with saturated brine, dried over anhydrous sodium sulfate, filtered, concentrated, and purified by reverse-phase column chromatography (acetonitrile/water = 30%-100%) to obtain compound 1c (8 g, yield 50.11%).
MS m/z(ESI):487.1[M-56]⁺.

### Step 3:

Compound 1c (8000 mg, 14.72 mmol) was dissolved in THF (100 mL), H₂O₂ (30% aqueous solution, 25.09 mL, 248 mmol) and an aqueous solution (20 mL) of lithium hydroxide monohydrate (926.51 mg, 22.08 mmol) were added, and reacted at room temperature for 2.5 hours. An aqueous solution (100 mL) of sodium bisulfite (2.09 g, 29.74 mmol) was added. 2N NaOH aqueous solution was added to adjust the pH to10, and washed twice with TMBE, 6M HCl was added to the aqueous phase to adjust the pH to 2-3, and extracted three times with TMBE, the organic phases were combined, and washed with saturated brine, dried over anhydrous sodium sulfate, filtered, concentrated, and purified by reverse-phase preparation (acetonitrile/water = 20%-100%) to obtain compound 1d (5650 mg, yield 99.88%).
MS m/z (ESI): 384.1 [M-H]⁻.

### Step 4:

Compound 1d (5650 mg, 14.70 mmol) was dissolved in TMBE (100 mL), NH₃/MeOH (7 M) (6 mL) was added, stirred at room temperature overnight, and concentrated to obtain a white solid(5900 mg). The solid was dissolved in 2-MeTHF (100 mL), O-tert-butyl-N,N'-diisopropylisourea (11781.18 mg, 58.81 mmol) was added, and reacted at 65°C overnight. Water was added, extracted with EtOAc, washed with saturated brine, dried over anhydrous sodium sulfate, filtered, concentrated, and purified by column chromatography (EtOAc/PE = 0-20%) to obtain compound 1e (5070 mg, yield 78.31%) as a colorless oil.
MS m/z(ESI):328.1[M-112]⁺.

### Step 5:

Compound 1e (5070 mg, 11.51 mmol) was dissolved in THF (50 mL), and Pd(dppf)Cl₂·CH₂Cl₂ (940.18 mg, 1.15 mmol), Cs₂CO₃(11253.29 mg, 34.54 mmol), and potassium vinyltrifluoroborate (97% purity, 4626.41 mg, 33.50 mmol) were added. After nitrogen protection, the tube was sealed and reacted at 80°C overnight, water was added, extracted with EtOAc, washed with saturated brine, dried over anhydrous sodium sulfate, filtered, concentrated, and purified by column chromatography (EtOAc/PE=0-20%) to obtain compound 1f (3960 mg, yield 88.76%) as a colorless oil. MS m/z(ESI):276.1[M-112]⁺.

### Step 6:

Compound 1f (3960 mg, 10.22 mmol) was dissolved in THF (60 mL), an aqueous solution (30 mL) of sodium periodate (4360.37 mg, 20.44 mmol) was added, OsO₄ (4% in water, 4 mL) was added, and reacted at room temperature overnight. The solid was filtered off, water was added, and extracted with EtOAc, the organic phases were combined, washed with saturated brine, dried over anhydrous sodium sulfate, filtered, concentrated, and purified by column chromatography (EtOAc/PE=0-30%) to obtain compound 1g (1140 mg, yield 28.64%) as a colorless oil.
MS m/z(ESI):234.1[M-156]⁺.

### Step 7:

Compound 1g (145 mg, 0.37 mmol) and 3-aminomethylpyridine (20 mg, 0.185 mmol) were dissolved in 5 mL MeOH, acetic acid (11.11 mg, 0.185 mmol) and 4A molecular sieve (20 mg) were added, and reacted at room temperature for 3 hours. Sodium cyanoborohydride (34.87 mg, 0.555 mmol) was added, and stirred at room temperature overnight. Filtered, 2N sodium hydroxide aqueous solution was added to adjust the pH to 8-9, the solvent was removed by rotary evaporation, purified by preparative high-performance liquid chromatography (MeOH/H₂O) to obtain compound 1h (19 mg, yield 12%) as a colorless oil.
MS m/z(ESI):855.5[M+H]⁺.

### Step 8:

Compound 1h (19.0 mg, 0.022 mmol) was dissolved in HCl/EtOAc (4M, 2 mL), and reacted at room temperature overnight. The reaction system was cooled using a dry ice-ethanol bath, purified water (5 mL) was slowly added to the reaction system, the system was further cooled until it became frozen, then freeze-dried directly to obtain the hydrochloride of compound 1 (11.2 mg, yield 78.1 %) as a white solid.
MS m/z(ESI):543.3[M+H]⁺.

### Example 2

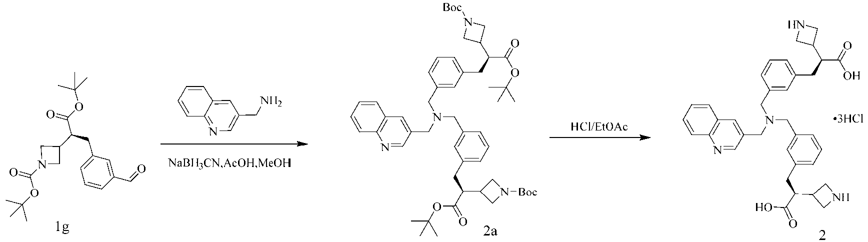

### Step 1:

Compound 1g (100 mg, 0.26 mmol) was dissolved in methanol (5 mL), 3-aminomethylquinoline (20.33 mg, 0.13 mmol) was added, five pellets of 4A molecular sieve were added, after stirring at room temperature for 1 hour, glacial acetic acid (46.25 mg, 0.77 mmol) and sodium cyanoborohydride (48.4 mg, 0.77 mmol) were added, stirred at room temperature overnight. After the reaction solution was filtered, purified by reverse-phase preparation (methanol/water = 80%-100%) to obtain compound 2a as a white solid.

### Step 2:

Compound 2a (70 mg, 0.077 mmol) was dissolved in HCl/EtOAc (4 M, 5 mL), reacted at 40°C overnight. Water (10 mL) was added, washed twice with EtOAc, the aqueous phase was freeze-dried to obtain the hydrochloride of compound 2 (53.29 mg, 98.15%).
MS m/z(ESI):593.4[M+H]⁺.

### Example 3

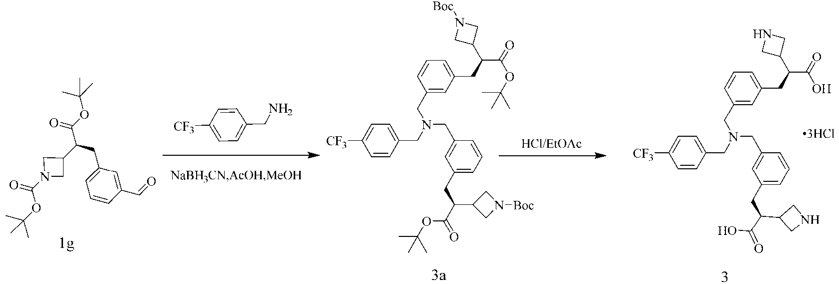

### Step 1:

Compound 1g (100 mg, 0.26 mmol) and p-trifluoromethylbenzylamine (22.48 mg, 0.13 mmol) were dissolved in 5 mL MeOH, acetic acid (7.71 mg, 0.13 mmol) and 4A molecular sieves (20 mg) were added, and reacted at room temperature for 3 hours. Sodium cyanoborohydride (24.2 mg, 0.385 mmol) was added, reacted at room temperature overnight. Filtered, 2N sodium hydroxide aqueous solution was added to adjust the pH to 8-9, the solvent was removed by rotary evaporation, purified by preparative high performance liquid chromatography (methanol/H₂O) to obtain compound 3a (35 mg, yield: 29.5%) as a colorless oil.

### Step 2:

Compound 3a (35 mg, 0.038 mmol) was dissolved in HCl/EtOAc (4 M, 2 mL), and reacted at room temperature overnight. The reaction system was cooled using a dry ice-ethanol bath, purified water (5 mL) was slowly added to the reaction system, and continue cooling until the system was frozen, then freeze-dried directly three times, gived the hydrochloride of compound 3 (27 mg, yield: 98.9%) as a white solid.
MS m/z(ESI):610.3[M+H]⁺.

### Example 4

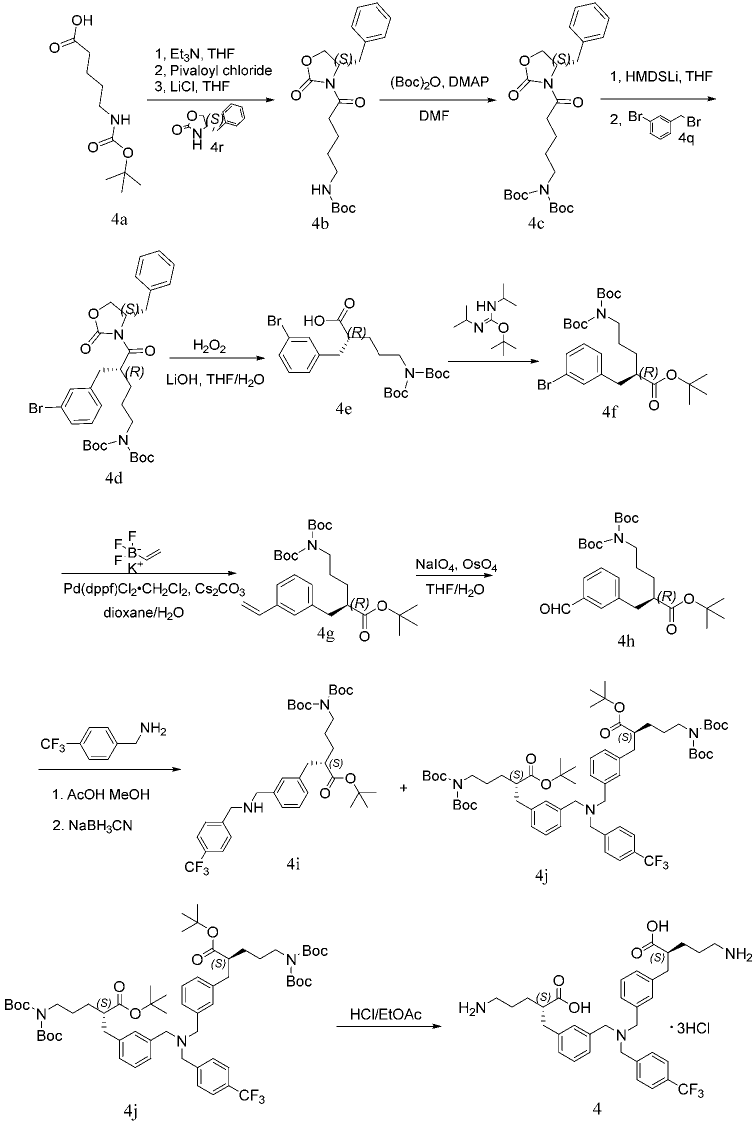

### Step 1:

Compound 4a (22.81 g, 105.00 mmol) was dissolved in anhydrous tetrahydrofuran (240 mL), under nitrogen protection and ice bath, triethylamine (25.30 g, 250.00 mmol) was added, and after stirring for 5 minutes, pivaloyl chloride (15.07 g, 125.00 mmol) was slowly added dropwise. After stirring at 10°C for 30 minutes, anhydrous lithium chloride (5.30 g, 125 mmol) and a solution of compound 4r (17.72 g, 100.00 mmol) in anhydrous tetrahydrofuran (240 mL) were added, and returned to room temperature and stirred overnight. 1 M HCl aqueous solution (300 mL) was added under water bath, after stirring for 1 minute and then allowed to stand to separate into layers, the upper organic phase was separated and washed with 1 M NaOH aqueous solution (300 mL), washed with saturated brine (300 mL), dried over anhydrous sodium sulfate, filtered and concentrated, purified by silica gel column (A/EtOAc=100/0-80/20, A:PE/DCM=1/1) to give a colorless oily liquid. The colorless oily liquid was dissolved in anhydrous tetrahydrofuran (50 mL) and concentrated to obtain compound 4b (26.00 g, yield 65.7%) as a colorless oily liquid.

### Step 2:

Compound 4b (26.00 g, 69.07 mmol) was dissolved in DMF (200 mL), triethylamine (20.97 g, 207.20 mmol) and DMAP (0.84 g, 6.91 mmol) were added under water bath, and Boc₂O (22.61 g, 103.60 mmol) was slowly added, sealed with a nitrogen balloon, after stirring at room temperature overnight, the reaction was monitored by TLC, which showed that the formation of half of the product and leaving the other half of the starting material unreacted. Triethylamine (13.97 g, 138.13 mmol) and Boc₂O (15.07 g, 40.03 mmol) were added, and further stirred overnight. Dilute with ethyl acetate (600 mL), washed with water (200 mL×3), concentrated, and purified by silica gel column (PE/EtOAc=100/0-84/16) to obtain compound 4c (24.00 g, yield 72.9%) as a pale yellow oily liquid, dissolved in anhydrous THF and concentrated twice, the product was directly used in the next step. Step 3:

Under nitrogen protection, compound 4c (9.70 g, 20.354 mmol) was added to anhydrous THF (80 mL), cooled to about -70 °C, LiHMDS/THF solution (1 M, 24.43 mL) was added dropwise, stirred for 1 hour, and the solution (40 mL) of compound 4q (6.10 g, 24.43 mmol) in anhydrous THF was added dropwise, warmed to 0 °C and stirred for 3 hours, after the reaction was monitored to completion by TLC, a saturated ammonium chloride aqueous solution (200 mL) was added at 0 ° C to quench the reaction, the organic phase was separated, and washed with saturated brine (200 mL), dried over anhydrous sodium sulfate, filtered and concentrated, and slurried with petroleum ether to obtain a white solid (8.60 g), the filtrate was purified by silica gel column obtain a white solid (2.00 g), combined two batches of product compound 4d, yielding a total of (10.60 g, yield 80%).

### Step 4:

Compound 4d (10.20 g, 15.80 mmol) was dissolved in THF (60 mL), hydrogen peroxide (2.687 mL, 23.70 mmol), LiOH (0.99 g, 23.70 mmol) aqueous solution (24 mL) were added under an ice bath, and stirred for 3 hours in an ice bath, the reaction was monitored to completion by TLC. An saturated NaHSO₃ aqueous solution (5 mL) was added dropwise under ice bath, returned to room temperature and stirred for 5 minutes, and the pH was adjusted to 2-3 using 1M HCl, extracted with ethyl acetate (100 mL×2), washed with saturated sodium chloride aqueous solution, concentrated, reversed (water/acetonitrile, 62% peak), concentrated, extracted with ethyl acetate (100 mL×2), dried over anhydrous sodium sulfate, filtered and concentrated to obtain compound 4e (6.00 g, yield 78%) as a colorless oily liquid.

### Step 5:

Compound 4e (6.00 g, 12.34 mmol) was dissolved in 2-methyltetrahydrofuran (50 mL), triethylamine (2.50 g, 24.67 mmol) was added, after stirring at room temperature for 5 minutes, O-tert-butyl-N,N'-diisopropylisourea (8.65 g, 43.17 mmol) was added, stirred at 65°C overnight under the protection of a nitrogen balloon, and cooled to room temperature, filtered, and the filtrate was concentrated, purified by silica gel column (PE/EtOAc=10/1) to obtain compound 4f (5.80 g, yield 86.67%) as a colorless oily liquid.

### Step 6:

Compound 4f (5.80 g, 10.69 mmol) was dissolved in a mixture of dioxane/water (30/10 mL), and potassium vinyl trifluoroborate (4.30 g, 32.07 mmol), CS₂CO₃ (10.45 g, 32.07 mmol), and Pd(dppf)Cl₂·CH₂Cl₂ (0.44 g, 0.535 mmol) were added under a water bath. The system was replaced with argon three times, stirred at 90°C overnight under argon protection, cooled, concentrated to remove most of the dioxane by rotary evaporation, diluted with ethyl acetate (100 mL), washed with water (50 mL), concentrated, and purified by silica gel column (PE/EtOAc=5/1) to obtain compound 4g (4.80 g, yield 91.67%) as a colorless oily liquid.

### Step 7:

Compound 4g (4.80 g, 9.80 mmol) was dissolved in a mixture of THF/water (60/30 mL), and O_{S}O₄ aqueous solution (0.98 mmol, 6.24 mL) was added under a water bath, after stirring for 15 minutes, sodium periodate (6.29 g, 29.41 mmol) was added under a water bath, and stirred at 35°C overnight. The reaction was monitored to completion by TLC, an saturated sodium thiosulfate aqueous solution (50 mL) was added under a water bath, after stirring for 5 minutes, concentrated to remove most of THF, and diluted with ethyl acetate (150 mL), washed with water (50 mL×2), concentrated, and purified by silica gel column (PE/EtOAc=100/0~86/14) to obtain compound 4h (3.6 g, yield 75%) as a colorless oily liquid.

### Step 8:

Compound 4h (100 mg, 0.20 mmol) was dissolved in methanol (10 mL), p-trifluoromethylbenzylamine (35.63 mg, 0.20 mmol) was added, 4A molecular sieves (20 mg, about 5 pellets) were added and stirred for 2 hours, acetic acid (20 uL) was added, and then sodium cyanoborohydride (63.91 mg, 1.02 mmol) was added, and stirred at 70°C under sealed conditions overnight. The solvent was removed by rotary evaporation, H₂O (5 mL) was added, and 2N NaOH aqueous solution was added to adjust the pH to 8-9, and the organic phase was extracted with ethyl acetate (5 mL×3), the organic phases were combined and concentrated by rotary evaporation, and purified by reverse-phase preparation (methanol/H₂O) to obtain compound 4i (40 mg, yield: 30.22%) as a white solid and compound 4j (50 mg, yield: 21.82%) as a white solid.

### Step 9:

Compound 4j (38 mg) was added to a reaction bottle, and EtOAc (2 mL) and 4N HCl/EtOAc (2 mL) were added, stirred for 2 hours, and the reaction system was cooled using a dry ice-ethanol bath, purified water (5 mL) was slowly added until it was frozen into ice, after extraction with EtOAc (3 mL), subjected to freeze-drying using a lyophilizer to obtain the hydrochloride of compound 4 (24.2 mg, yield: 97.89%) as a white flocculent solid.
MS m/z(ESI):614.3[M+H]⁺.

### Example 5

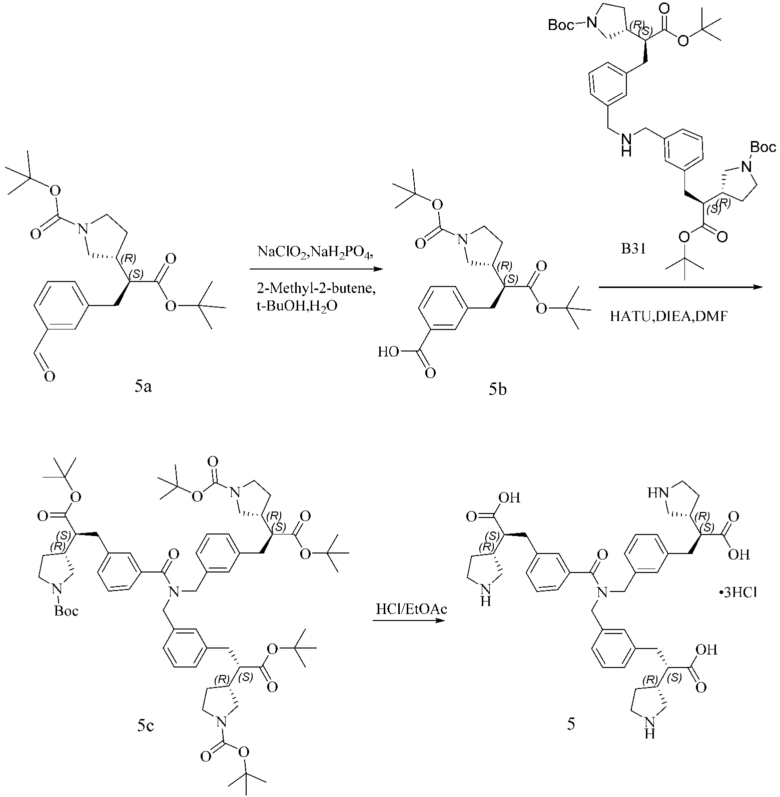

### Step 1:

The reactant 5a (5 g, 12.39 mmol) was added to tert-butyl alcohol (50 mL), water (50 mL), and NaClO₂ (11.21 g, 123.91 mmol) and stirred to dissolve. NaH₂PO₄ (8.92 g, 74.35 mmol) and 2-methyl-2-butene (10 mL) were added, stirred at 25°C overnight. The solvent was removed from the system by rotary evaporation, EtOAc (80 mL) was added, and the liquid was separated, and the aqueous phase was extracted once with EtOAc (40 mL), the organic phases were combined, washed with saturated sodium chloride solution (40 mL), separated, and the organic phase was dried. Filtered, the filtrate was concentrated by rotary evaporation, and mixed with silica gel, and purified by column chromatography (EtOAc/PE 0-50%), and compound 5b (4.3 g, yield 82.72%) was obtained by rotary evaporation.
MS m/z(ESI):320.3[M-100+H]⁺.

### Step 2:

The reactant 5b (700 mg, 1.67 mmol) was dissolved in DMF (5 mL), and HATU (1008 mg, 2.65 mmol) and DIEA (685 mg, 5.3 mmol) were added, after stirring at room temperature for 15 minutes, a DMF solution (5 mL) of B31 (1400 mg, 1.77 mmol) was added, and reacted at 30°C overnight. Water was added, extracted with EtOAc, washed with saturated brine, dried over anhydrous sodium sulfate, filtered, concentrated, and purified by reverse-phase preparation (methanol/water) to obtain compound 5c (1.5 g, yield 75.32%) as a solid.
MS m/z(ESI):497.4[M/2-100]⁺.

### Step 3:

Compound 5c (770 mg, 0.645 mmol) was dissolved in 4M HCl/EtOAc, and reacted at 40°C overnight. Water was added, washed twice with EtOAc, the aqueous phase was lyophilized to obtain the hydrochloride of compound 5 (510 mg, yield 94.76%).
MS m/z(ESI):726.5[M+H]⁺.

### Example 6

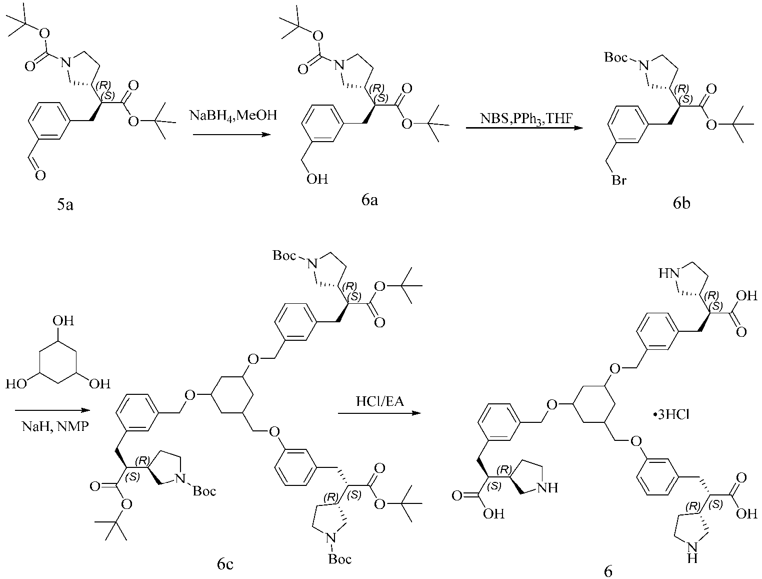

### Step 1:

Compound 5a (2000 mg, 4.96 mmol) was dissolved in methanol (100 mL), and NaBH₄ (335.1 mg, 9.91 mmol) was added at -20°C, and the mixture was reacted at room temperature overnight. After concentration, a saturated sodium bicarbonate aqueous solution was added, and the mixture was extracted with EtOAc, washed with saturated brine, dried over anhydrous sodium sulfate, filtered, and concentrated to obtain compound 6a (2000 mg, yield 99.5%) as a colorless oil.
MS m/z(ESI):306.2[M+H-100]⁺.

### Step 2:

Compound 6a (1800 mg, 4.44 mmol) was dissolved in THF (100 mL), and PPh₃ (2310 mg, 8.81 mmol) and NBS (900 mg, 5.06 mmol) were added at -20°C, and reacted for 3 hours. After filtration, water (10 mL) was added to the filtrate, and the mixture was extracted three times with EtOAc, the organic phases were combined, washed with saturated brine, filtered, concentrated, and purified by column chromatography (EtOAc/PE=0-20%) to obtain compound 6b (1300 mg, yield 62.53%) as a colorless oil.
MS m/z(ESI):356.1[M-112]⁺.

### Step 3:

Compound 6b (620.27 mg, 1.32 mmol) and 1,3,5-cyclohexanetriol (50 mg, 0.38 mmol) were dissolved in N-methylpyrrolidone (5 mL), sodium hydride (90.80 mg, 3.78 mmol) was added, replaced with nitrogen three times, and reacted at room temperature for 16 hours. Water (2 mL×3) was added for washing, the organic phase was extracted with ethyl acetate (3 mL×3), the organic phases were combined, dried by rotary evaporation, and purified by reverse-phase preparation (methanol/H₂O) to obtain compound 6c (90 mg, yield: 18.3%) as a light yellow solid.
MS m/z(ESI):520.0[(M-256)/2+H]⁺.

### Step 4:

Compound 6c (50 mg, 0.03 mmol) was dissolved in HCl/EtOAc (4 M, 3 mL), and reacted at room temperature for 16 hours. Water (3 mL) was added to the reaction solution, and the liquid was separated, and the aqueous phase was washed with ethyl acetate (3 mL×3), and the aqueous phase was lyophilized to obtain the hydrochloride of compound 6 (19.75 mg, yield: 54.68%) as a light yellow solid.
MS m/z(ESI):827.1[M+H]⁺.

### Example 7

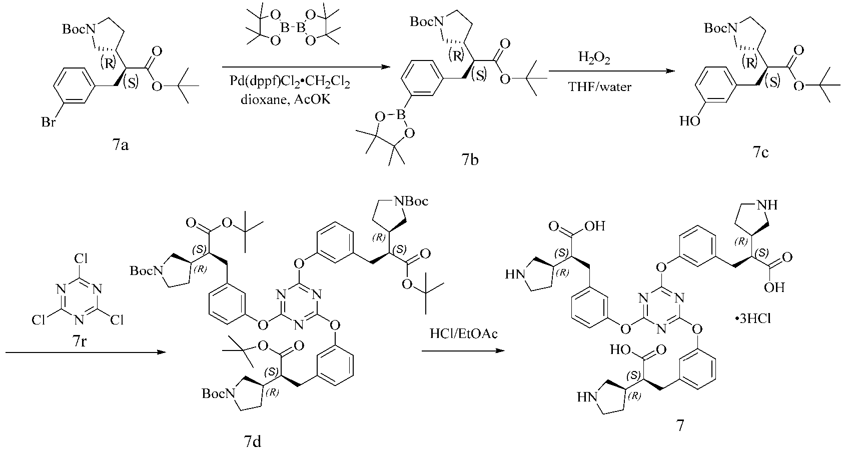

### Step 1:

Compound 7a (3.00 g, 6.60 mmol) was dissolved in dioxane (26 mL), and pinacol borate (2.51 g, 9.90 mmol), potassium acetate (1.29 g, 13.20 mmol) and Pd(dppf)Cl₂·CH₂Cl₂ (0.27 g, 0.33 mmol) were added. The mixture was replaced with argon three times, and stirred at 90°C overnight under argon protection. The disappearance of starting material and the formation of new spots were monitored by TLC. After cooling, concentrated, diluted with ethyl acetate (50 mL), washed with water (50 mL), and concentrated to obtain compound 7b (3.31 g, yield 100%) as a red liquid.

### Step 2:

Compound 7b (3.31 g, 6.60 mmol) was dissolved in THF (50 mL), and a solution of NaOH (0.79 g, 19.80 mmol) in water (10 mL) was added. H₂O₂ (7.50 mL, 66.00 mmol) was slowly added dropwise under ice bath. After the addition was completed, the mixture stirred at 25°C overnight. Saturated sodium thiosulfate aqueous solution (20 mL) was added under water bath to quench the hydrogen peroxide,the pH was adjusted to 3 with 1 M hydrochloric acid, concentrated by rotary evaporation and most of the THF was removed, ethyl acetate (50 mL×2) was added for extracting, the organic phases were combined, washed with water (50 mL), concentrated, and passed through a silica gel column (PE/EtOAc=1/0-3/1) to obtain compound 7c (2.08 g, yield 80%) as a white solid.

### Step 3:

Compound 7c (400 mg, 1.02 mmol) was dissolved in anhydrous THF (10 mL), and 4A molecular sieves (50 mg) were added under the protection of an argon balloon, stirred at room temperature for 10 minutes, and then placed in an ice bath, and sodium hydride (40.88 mg, 1.02 mmol) was added, returned to room temperature and stirred for 20 minutes, compound 7r (47.10 mg, 0.255 mmol) was added under an ice bath, stirred at 40°C overnight. The formation of new spots was monitored by TLC. Saturated aqueous ammonium chloride solution (20 mL) was added under an ice bath, and extracted with ethyl acetate (20 mL×2), the organic phases were combined, washed with water (50 mL×1), concentrated, and passed through a silica gel column (PE/EtOAc=3/1) to obtain compound 7d (200.00 mg, yield 15.66%) as a white solid.

### Step 4:

Compound 7d (40.00 mg, 0.03 mmol) was added to a single-necked flask, hydrochloride/ethyl acetate (5 mL) was added, and stirred at room temperature overnight, deionized water (10 mL) was added at -20°C, the aqueous phase was separated, and the aqueous phase was back-extracted with ethyl acetate (10 mL×3), the aqueous phase was lyophilized, deionized water (10 mL) was added to dissolve, and then lyophilized to obtain the hydrochloride of compound 7 (27.03 mg, yield 94.88%) as a yellow solid.

### Example 8

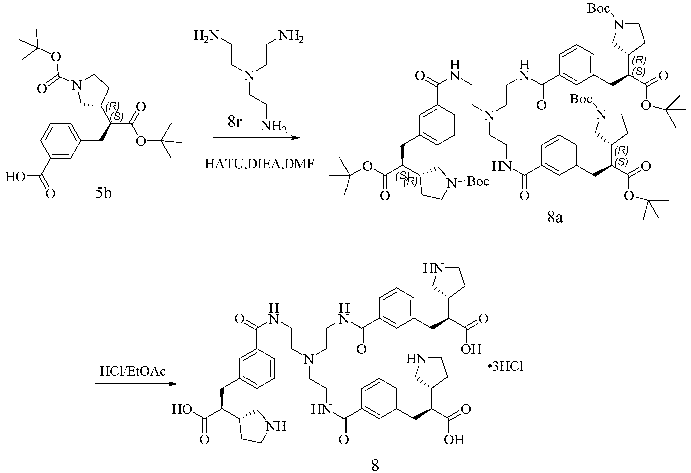

### Step 1:

Compound 5b (94.67 mg, 0.23 mmol) was dissolved in DMF (5 mL), and HATU (104 mg, 0.27 mmol) and DIEA (53.03 mg, 0.41 mmol) were added, after reacting at room temperature for 15 minutes, compound 8r (10 mg, 0.068 mmol) was added and reacted at room temperature overnight. Water was added, extracted with EtOAc, washed with saturated brine, dried over anhydrous sodium sulfate, filtered, and concentrated. After preparation (methanol/water), compound 8a (87.75 mg, 95%) was obtained.
MS m/z(ESI):474.4[M/2-200]⁺.

### Step 2:

Compound 8a (87.75 mg, 0.065 mmol) was dissolved in 4M HCl/EtOAc (5 mL), reacted at 40°C overnight. Water (10 mL) was added, washed twice with EtOAc, the aqueous phase was lyophilized to obtain the hydrochloride of compound 8 (58.48 mg, yield 87.57%).
MS m/z(ESI):883.5[M+H]⁺.

### Example 9

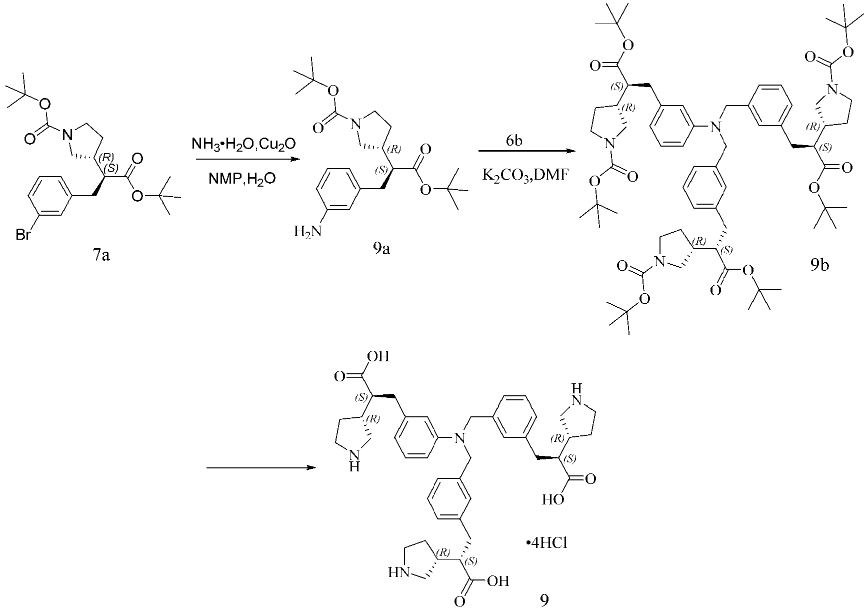

### Step 1:

The reactant 7a (1.2 g, 2.64 mmol) was added to the reaction bottle, NMP (2 mL) was added, and stirred. Ammonia water (5 mL) was added and stirred. Cuprous oxide (0.38 g, 2.65 mmol) and water (2 mL) were added and continued stirring. Warm up to 80°C and stirred overnight. Water (20 mL) was added and stirred, extracted with EtOAc (20 mL), and phases were separated, the organic phase was dried by rotary evaporation, mixed with silica gel. Purified by column chromatography PE/EtOAc (80:20) to obtain the product, the product was dried by rotary evaporation to obtain compound 9a (726 mg, yield 70.4%) as a solid.

### Step 2:

Compound 6b (200 mg, 0.51 mmol) was dissolved in DMF (7 mL), K₂CO₃ (212.35 mg, 1.54 mmol) and compound 9a (263.89 mg, 0.56 mmol) were added, reacted at 60°C overnight. Compound 9b (135 mg, yield 22.62%) was obtained by preparative high-performance liquid chromatography (MeOH/H₂O).

### Step 3:

Compound 9b (120 mg, 0.10 mmol) was dissolved in 4M HCl/EtOAc (7 mL) , reacted at 40°C for 4 hours. Water was added, washed twice with EtOAc, the aqueous phase was lyophilized to obtain the hydrochloride of compound 9 (64 mg, yield 73.77%).
MS m/z(ESI):698.4[M+H]⁺.

### Example 10

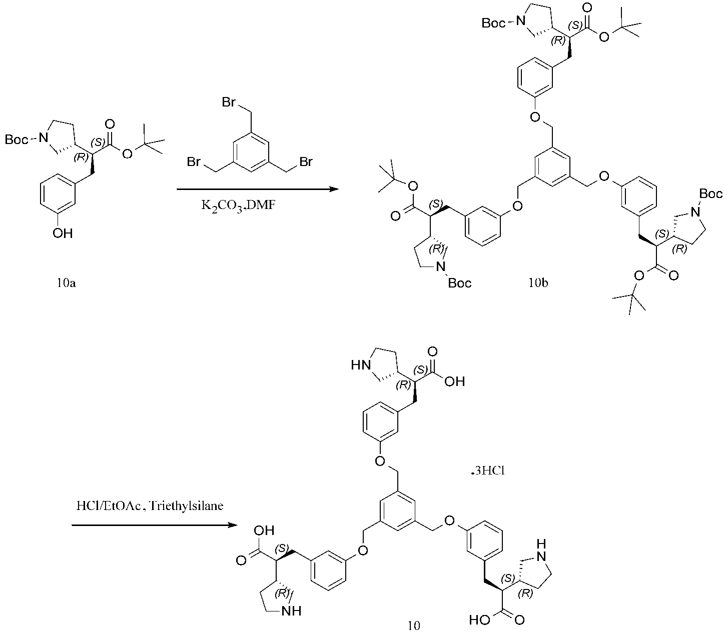

### Step 1:

1,3,5-tri(bromomethyl)benzene (37 mg, 0.10 mmol) and compound 10a (162 mg, 0.41 mmol) were dissolved in DMF (3 mL), potassium carbonate (201 mg, 0.61 mmol) was added, stirred at 60°C overnight, filtered, and the filtrate was purified by reverse-phase preparative chromatography (MeOH/H₂O) to obtain compound 10b (70 mg, yield 52%) as a white solid.

### Step 2:

Compound 10b (70 mg, 0.054 mmol) was dissolved in ethyl acetate (1 mL), triethylsilane (0.1 mL) and HCl/EtOAc (4 mol/L, 3 mL) were added, stirred at room temperature for 2 hours, and concentrated to obtain the solid which was washed with ethyl acetate three times, pure water (10mL) was added for dissolving, and lyophilized to obtain the hydrochloride of compound 10 (36.18 mg, yield 72%) as a white solid.

### Example 11

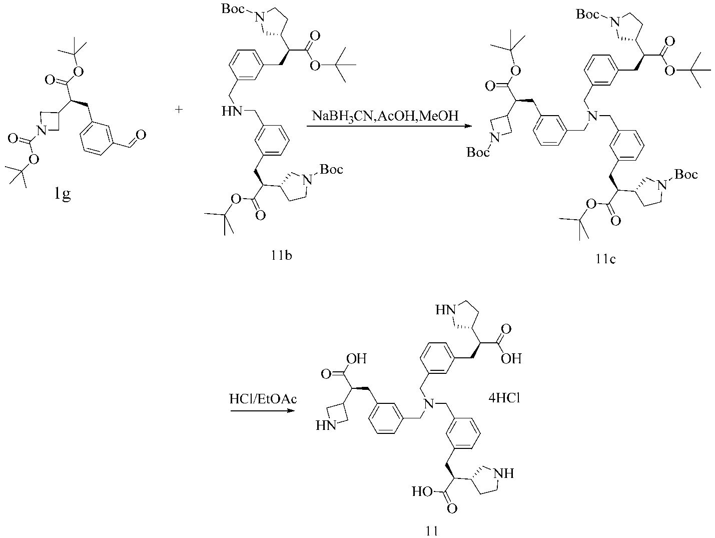

### Step 1:

Compound 1g (60 mg, 0.154 mmol) was dissolved in methanol (5 mL), 11b (110 mg, 0.139 mmol) and AcOH (37 mg, 0.616 mmol) were added, 5 pellets of 4A molecular sieves were added to remove water, and reacted at room temperature for 1 hour, then AcOH (37 mg, 0.616 mmol) and NaBH₃CN (21 mg, 0.621 mmol) were added, and reacted at 70°C overnight. After filtering off the solid, the filtrate was concentrated. Compound 11c (70mg, yield 39%) was obtained as a colorless oil by preparative high-performance liquid chromatography (MeOH/H₂O).
MS m/z(ESI):533.5[M/2-50]⁺.

### Step 2:

Compound 11c (70 mg, 0.26 mmol) was dissolved in 4M HCl/EtOAc (5 mL), reacted at room temperature overnight. The solid was produced, water was added, washed twice with EtOAc, and the aqueous phase was lyophilized twice to obtain the hydrochloride of compound 11 (48 mg, yield 95%).
MS m/z(ESI):697.2[M+H]⁺.

### Comparative Example 1

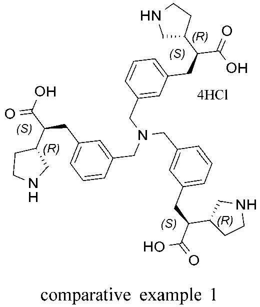

Comparative compound 1 can be synthesized with reference to patent WO2020/247429A1.

### BIOLOGICAL TEST EVALUATION

### Test Example 1: Using SPR to detect the affinity of the compounds of the present invention to Apo(a)

1. Experimental purpose: The purpose of the test is to test the affinity of the compounds of the present invention to Apo(a)
2. Experimental instruments and reagents:
   Biomolecular interaction analyzer (Biacore 8K),
   Apolipoprotein (a) was synthesized by Hangzhou Haoyang Biotechnology Co., Ltd. NHS was purchased from Cytiva.
3. Experimental methods:
   Apolipoprotein(a) was covalently linked to a Series S Sensor Chip CM5 in a Biacore 8K instrument. The activator was prepared by mixing 400 mM EDC and 100 mM NHS before injection. The CM5 sensor chip was activated with the activator for 420 seconds at a flow rate of 10 µL/min. 60 µg/mL apolipoprotein(a) was dissolved in 10 mM NaAc solution (pH 4.5), and injected into the Fc2 sample channel at a flow rate of 10 µL/min to achieve an immobilization level of around 10,000 RU. It was inactivated for 420 seconds with 1 M ethanolamine hydrochloride-sodium hydroxide at a flow rate of 10 µL/min. The reference channel Fc1 was blocked using the same procedure as that used for Fc2, but without performing the protein injection step. The compound was diluted to 100 nM with running buffer (1×PBS containing 0.005% Tween-20, pH 7.4), injected into channel Fc1-Fc2 at a flow rate of 30 µL/min, bound for 90 seconds, and dissociated for 210 seconds. Both the binding and dissociation processes were carried out in the running buffer. 3M magnesium chloride was injected at a flow rate of 20 µL/min in the running buffer for 30 seconds to regenerate the chip. The affinity constant KD reflects the size of the interaction binding ability, when the concentration of the compound is at KD, the equilibrium signal Req is half of Rmax. The results show that the compounds of the present invention has a strong binding ability with human Apo(a) protein.

### Test Example 2: Using BLI to detect of the affinity of the compounds of the present invention to Apo(a)

1. Experimental purpose: The purpose of this test is to test the affinity of the compounds of the present invention to Apo(a)
2. Experimental instruments and reagents:
   Molecular interaction analyzer (ForteBio Octet red 96e),
   SA sensor (ForteBio),
   96-well plates were purchased from Greine,
3. Experimental methods:
   Prepared two 96-well plates, one as a sample plate and another as a pre-wett plate, curing buffer was added to a 200µL well of the pre-wet plate, and the SA sensor was pre-wetted for at least ten minutes. All reagents and samples were added to another black sample plate, after the sample addition was completed, the sensor plate and the sample plate were placed into the ForteBio Octet red 96e instrument. The program was set up in sequence for detection. The experimental temperature was set to 30°C and the acquisition frequency was set at 5.0Hz. The biosensor was run in 1 ×PBS, pH7.4, 0.02% Tween-20, 0.1% BSA in the first column for 60 seconds, which was the baseline step. Biotinylated apolipoprotein (a) was diluted to 40µg/ml with 1×PBS, pH7.4, 0.02% Tween-20, 0.1% BSA, and added to the second column, where it was run for a period of time until the amount of curing on the sensor reaches 2.0nm. The biosensor was run in 1×PBS, pH7.4, 0.02% Tween-20, 0.1% BSA in the third column for 60 seconds, which was the baseline step. The compound was diluted to 100 nM with 1×PBS, pH7.4, 0.02% Tween-20, 0.1% BSA, and added to the fourth column, where the sensor was run for 60 seconds. The sensor was run in 1×PBS, pH7.4, 0.02% Tween-20, 0.1% BSA in the third column for 120 seconds, during this process, the compound dissociated from the sensor. The affinity constant KD reflects the size of the interaction binding ability. When the concentration of the analyte is at KD, the equilibrium signal Req is half of Rmax. The results show that the compounds of the present invention has a strong binding force with human Apo(a) protein.

| Example | KD (nM) |
|---|---|
| Example 5 | 10.9 |
| Example 6 | 19.9 |
| Example 7 | 8.06 |
| Example 8 | 18.7 |
| Example 10 | 25.3 |
| Example 11 | 14.2 |

### Test Example 3: Mice PK

1. Experimental purpose: Purpose of this test is to test the PK of the compounds of the present invention in mice
2. Experimental animals:
   Mouse (C57BL-6J),
3. Experimental methods:
   C57BL-6J mice were housed with a standard light cycle (12 hours light/12 hours dark), at a room temperature of 18-26°C and a relative humidity of 40-70%, with free access to water and normal diet. Five days prior to the study, mice were randomly assigned to groups (n=3 per group) based on their body weight for the study. The administration volume was calculated according to the body weight of the animals on the day of administration, and the administration volume was 10 mL/kg, the single oral gavage administration was performed at a dose of 10 mg/kg, and the solvent was 1% HEC and 0.25% Tween 80 in Water. Blood was collected from the submandibular vein or other suitable veins at 0.5h, 1h, 2h, 4h, 6h, 8h, 12h, 24h, 48h, and 72h after administration. Each sample was collected at about 0.03mL per time point. After blood sample collection, placed on ice, and within 1 hour, centrifuged at 4°C, 6800g, for 10 minutes to separate plasma for blood drug concentration detection.

### Test Example 4: In vivo Lp(a) inhibition in cynomolgus monkeys

1. Experimental purpose: The purpose of this test is to test the inhibitory effect of the compounds of the present invention on Lp(a) in cynomolgus monkeys
2. Experimental instruments and reagents:
   Fully automatic biochemical analyzer (Hitachi 7600 model),
3. Experimental methods:
   The cynomolgus monkeys were housed with a standard light cycle (12 hours light/12 hours dark), at a room temperature of 18-26°C and a relative humidity of 40-70%, with drinking water provided continuously for 24 hours, and feed was provided to the animals twice daily, in the morning (about 10:30) and in the afternoon (about 15:00). Five days prior to the study, the cynomolgus monkeys were randomly assigned to groups (n=3/group) based on their body weight and baseline serum Lp(a) concentration for the study. The administration volume was calculated according to the body weight of the animals on the first day of administration, and the administration volume was 5 mL/kg, administered once daily by oral gavage for 5 consecutive days, and the dosage was 3 mg/kg, and the solvent was 1% HEC and 0.25% Tween 80.

The adaptation period (after fasting overnight), before administration on Day 1, and 8 hours after administration on Day 5. 1 mL of whole blood was collected from the animal's cephalic vein or saphenous vein, placed at room temperature for 30 minutes, and then centrifuged at 4°C, 3500 rpm, for 10 minutes to separate the serum. The serum Lp(a) level was detected using a fully automatic biochemical analyzer. By setting the average Lp(a) level before administration as 0% inhibition, the percentage of Lp(a) reduction in each group was determined. The results confirmed that the compounds of the present invention have a good effect in reducing plasma Lp(a) levels in vivo. The in vivo Lp(a) inhibition rate of the compounds of the present invention in cynomolgus monkeys was shown in the following table:

| Example | Lp(a) inhibition rate (%) |
|---|---|
| Comparative Example 1 | 32.87±17.30 |
| Example 5 | 50.23±20.41 |

### Test Example 5: Exposure determination in beagle dogs

1. Experimental purpose: The purpose of this test is to test the exposure of the compound in beagle dogs
2. Experimental methods:
Beagle dogs were housed with a standard light cycle (12 hours light/12 hours dark), at a room temperature of 18-26°C and a relative humidity of 40-70%, with free access to water and normal diet. Five days prior to the study, the beagle dogs were randomly assigned to groupsbased on their body weight for the study, including an oral administration group (n=3/group).The administration volume was calculated according to the weight of the animals on the day of administration, and the administration volume was 5 mL/kg, the single oral gavage administration was performed at a dose of 4.5 mg/kg, and the solvent was 1% HEC and 0.25% Tween 80 in Water. Blood was collected from the forelimb vein or other suitable veins at 0.5h, 1h, 2h, 4h, 6h, 8h, 12h, 24h, 48h, 72h and 96h after administration. About 1 mL of each sample was collected, K2-EDTA anticoagulated, placed on ice after collection, and within 1 hour, centrifuged to separate plasma (centrifugation conditions: 2200g, 10 minutes, 2-8°C) for blood drug concentration detection. Exposures in beagle dogs were calculated as shown in the following table:

| Example | AUC_{(0-∞)} (h*ng/mL) |
|---|---|
| Comparative Example 1 | 37435.29 |
| Example 5 | 118713.17 |

### Test Example 6: Bioavailability determination in cynomolgus monkeys

1. Experimental purpose: The purpose of this test is to test the bioavailability of the compound in cynomolgus monkeys
2. Experimental methods:
The cynomolgus monkeys were housed with a standard light cycle (12 hours light/12 hours dark), at a room temperature of 18-26°C and a relative humidity of 40-70%, with free access to water and normal diet. Five days prior to the study, the cynomolgus monkeys were randomly assigned to an intravenous administration group and an oral administration group (n=2/group) on their body weight for the study.The administration volume was calculated according to the body weight of the animals on the day of administration, and the administration volume was 2 mL/kg, the single intravenous administration was performed at a dose of 1 mg/kg, and the solvent was 5% DMSO, 5% Solutol, and 90% Saline. Blood was collected from the forelimb vein or other suitable veins at 0.083h, 0.25h, 0.5h, 1h, 2h, 4h, 6h, 8h, 12h, 24h, 48h and 72h after administration. The administration volume was calculated according to the weight of the animals on the day of administration, and the administration volume was 5 mL/kg, the single oral gavage administration was performed at a dose of 7.5 mg/kg, and the solvent was 1% HEC and 0.25% Tween 80 in Water. Blood was collected from the forelimb vein or other suitable vein at 0.5h, 1h, 2h, 4h, 6h, 8h, 12h, 24h, 48h and 72h after administration. About 1mL of each sample was collected, K2-EDTA anticoagulated, placed on ice after collection, and within 1 hour, centrifuged to separate plasma (centrifugation conditions: 2200g, 10 minutes, 2-8°C) for blood drug concentration detection. The bioavailability F was calculated as shown in the following table:

| Example | bioavailability F (%) |
|---|---|
| Comparative Example 1 | 10.20 |
| Example 5 | 16.77 |

### Test Example 7: hERG Experiment

1. Experimental purpose: The purpose of this test is to test the inhibition rate of the compounds on the hERG potassium channel
2. Experimental methods:
   The hERG current was recorded using the whole-cell patch clamp technique. A suspension of HEK-293-hERG cell was added to a small culture dish and placed on an inverted microscope stage.

After the cells adhered to the dish, extracellular fluid was perfused at a flow rate of 1-2 mL/minute. The glass microelectrode was pulled in two steps by a microelectrode puller, after the electrode was filled with internal fluid, its water resistance was 2-5 MΩ. After establishing the whole-cell recording mode, the holding potential was maintained at -80 mV. A depolarizing voltage to +60 mV was applied for 850 ms, and then followed by repolarization to -50 mV for 1275 ms to elicit the hERG tail current. This set of pulse programs was repeated every 15 seconds throughout the experiment. After the current stabilized, the test compound was administered via continuous extracellular perfusion from low concentration to high concentration. Starting from a low concentration, perfusion was continued until the drug effect was stable, and then the next concentration was perfused. The inhibition rate of the compound on the hERG potassium channel was calculated as shown in the following table:

| Example | inhibition rate (%) | | |
|---|---|---|---|
| | 0.3 µM | 1 µM | 3 µM |
| Comparative Example 1 | 7.37 | 24.62 | 29.16 |
| Example 5 | -0.06 | 3.47 | 3.67 |

### Test Example 8: Salmonella typhimurium reverse mutation test

The revived strains or a single colony from the master plate were inoculated into the broth, and cultured in a thermostat gas bath vibrator at 100 to 120 rpm and 37±1°C for 10 to 16 hours, marked and stored for later use. The bottom agar medium (containing an appropriate amount of agar, Vogel-Bonner buffer, 20% glucose solution and 20% magnesium sulfate solution) was poured into a six-well plate, approximately 20mL-25mL into each well, and allowed to cool and solidify naturally, marked each well and stored for later use.

Three wells were set up in each group for parallel experiments. The top agar medium was sterilized under high pressure and kept warm at about 65°C. In each test tube, 0.1mL of negative control (DMSO) or positive control or test sample, 0.1mL of bacterial solution suspension, 0.5mL of phosphate buffer (0.2M PBS) (-S9) or 0.5mL of S9 mixed solution (+S9) and 2.5mL top culture medium were added, after vortex mixing, and then quickly and evenly spread on the culture dish which was already spreaded with the bottom agar medium. After natural cooling and solidification, the culture dish was inverted and placed in a 37°C incubator for 48 to 72 hours. After culturing for 48 to 72 hours, the culture dish was taked out, and the number of revertant colonies in each well was counted, the growth background was observed under a microscope to determine whether there was antibacterial or bactericidal activity, and the experimental results were recorded. The mutagenicity of the compounds of the present invention in various strains was shown in the following table:

| Example | Strain | Metabolic Activation | mutagenicity | | | | |
|---|---|---|---|---|---|---|---|
| | | | 62µg/ dish | 185µg/ dish | 556µg/ dish | 1667µg/ dish | 5000µg/ dish |
| Comparative Example 1 | TA98 | + | - | - | + | + | + |
| | | - | - | + | + | + | + |
| | TA1537 | + | - | - | - | - | - |
| | | - | - | - | - | - | - |
| | TA100 | + | - | - | - | - | - |
| | | - | - | - | - | - | - |
| | TA102 | + | - | - | - | - | - |
| | | - | - | - | - | - | - |
| | TA1535 | + | - | - | - | - | - |
| | | - | - | - | - | - | - |
| Example 5 | TA98 | + | - | - | - | - | - |
| | | - | - | - | - | - | - |
| | TA1537 | + | - | - | - | - | - |
| | | - | - | - | - | - | - |
| | TA100 | + | - | - | - | - | - |
| | | - | - | - | - | - | - |
| | TA102 | + | - | - | - | - | - |
| | | - | - | - | - | - | - |
| | TA1535 | + | - | - | - | - | - |
| | | - | - | - | - | - | - |

| | | | | | | | |
|---|---|---|---|---|---|---|---|
| Note: **In** the "Metabolic Activation" column:   + indicates the addition of S9 mixture;   - indicates no addition of S9 mixture. In the "Mutagenicity" column:   + indicates a positive mutagenic result;   - indicates a negative mutagenic result. | | | | | | | |

## Claims

1. A compound of formula I, formula II, formula III, or formula IV, or a pharmaceutically acceptable salt thereof, wherein the structure of the compound of formula I, formula II, formula III, or formula IV is as shown below: 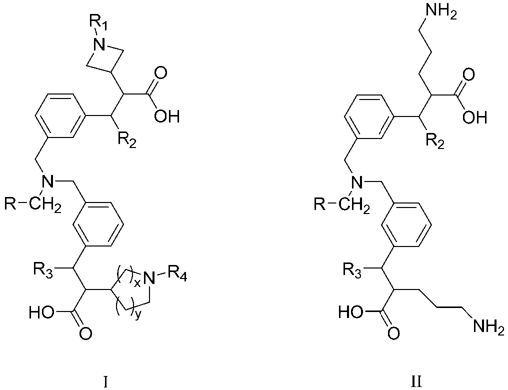 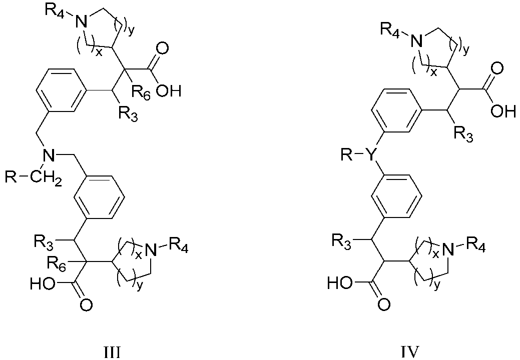 wherein,
R is selected from aryl or heteroaryl, which may be further substituted;
R₁, R₂, R₃ and R₄ are each independently selected from H and alkyl, wherein the alkyl may be further substituted;
R₆ is each independently selected from C1-6 alkyl; preferably methyl;
Y is a trivalent group, wherein the trivalent group is selected from 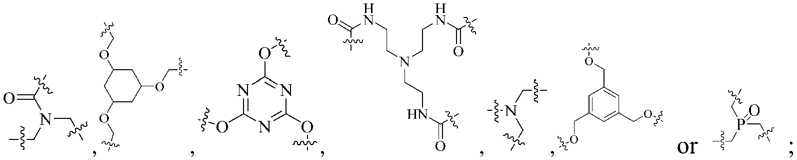
x is 1 or 2, preferably 1; and
y is 0 or 1.

2. The compound or the pharmaceutically acceptable salt thereof according to claim 1, wherein the compound of formula IV is a compound of formula IV-1, formula IV-2, formula IV-3, or formula IV-4:

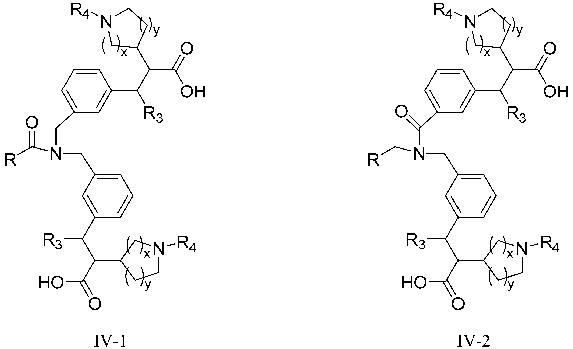

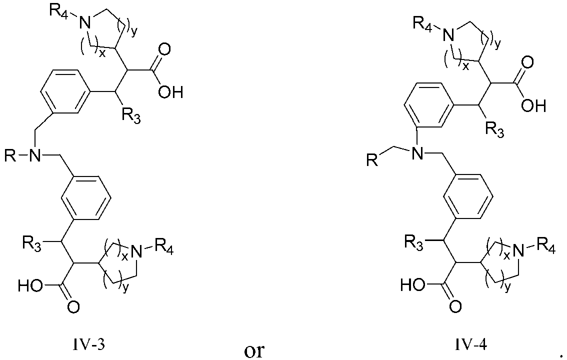

3. The compound or the pharmaceutically acceptable salt thereof according to any one of claims 1 to 2, wherein R is selected from C6-20 aryl, preferably C6-14 aryl, more preferably C6-12 aryl, which may be further substituted.

4. The compound or the pharmaceutically acceptable salt thereof according to any one of claims 1 to 2, wherein R₁, R₂, R₃ and R₄ are each independently selected from H and C1-6 alkyl, preferably H or methyl.

5. The compound or the pharmaceutically acceptable salt thereof according to claim 3, wherein R is selected from phenyl, benzo[d][1,3]dioxole, tetrahydroquinoline, tetrahydroisoquinoline, pyridine, quinoline or isoquinoline, which may be further substituted.

6. The compound or the pharmaceutically acceptable salt thereof according to claim 5, wherein R is selected from the following groups: 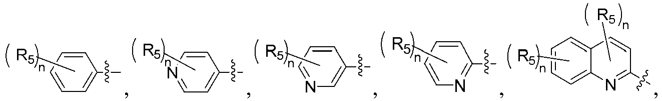 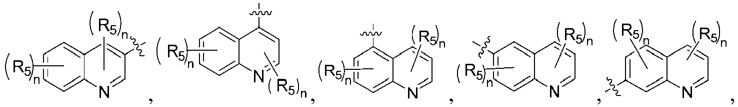 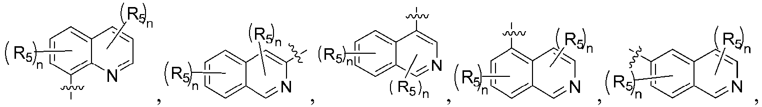 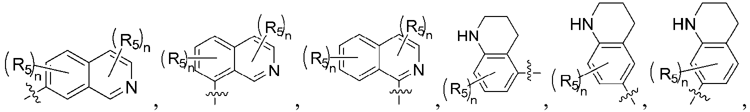 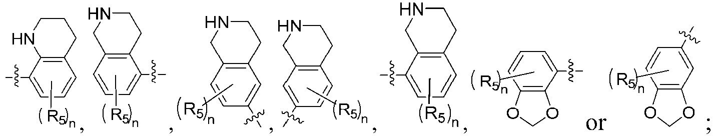
wherein, n is 0, 1, 2 or 3;
R₅ is each independently selected from hydrogen, alkyl, haloalkyl, alkoxy, haloalkoxy, alkylamino, alkylcarbonylamino, halogen, hydroxy, nitro, cyano, cycloalkyl, aryl or heteroaryl; preferably alkyl, haloalkyl, alkoxy, haloalkoxy, alkylamino, alkylcarbonylamino or halogen; and
the R₅ may be located at one, two, or three positions selected from the 1st, 2nd, 3rd, 4th, 5th, 6th, 7th and 8th positions of the aforementioned aryl or heteroaryl.

7. The compound or the pharmaceutically acceptable salt thereof according to claim 6, wherein R₅ is each independently selected from C1-6 alkyl, C3-6 cycloalkyl, C1-6 haloalkyl, C1-6 alkoxy, C1-6 haloalkoxy, C1-6 alkylcarbonylamino, halogen or

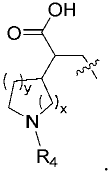

8. The compound or the pharmaceutically acceptable salt thereof according to claim 7, wherein R₅ is each independently selected from trifluoromethyl, 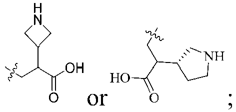 wherein, preferably 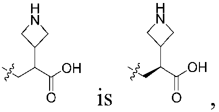 preferably

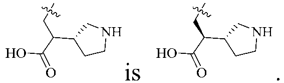

9. The compound or the pharmaceutically acceptable salt thereof according to claim 6, wherein R is selected from the following groups:

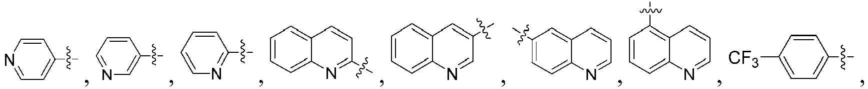

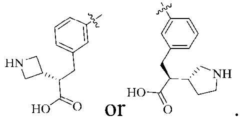

10. The compound or the pharmaceutically acceptable salt thereof according to claim 1, wherein the compound is selected from the following compounds: 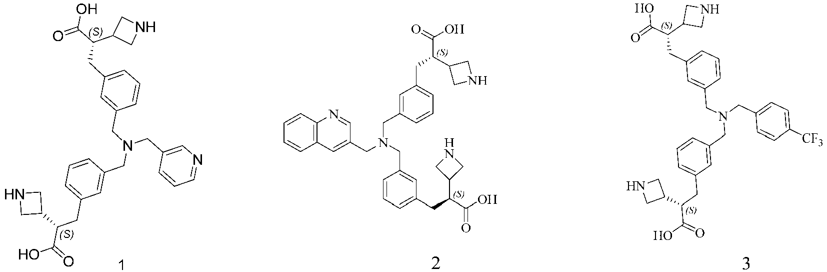 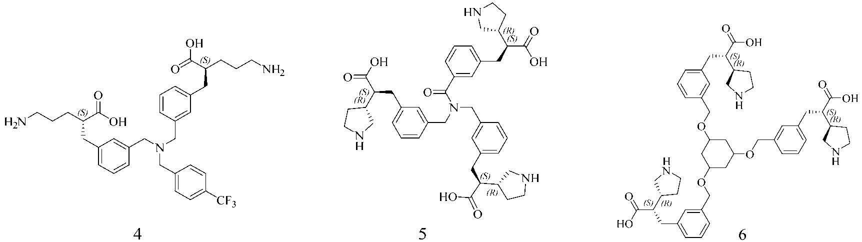 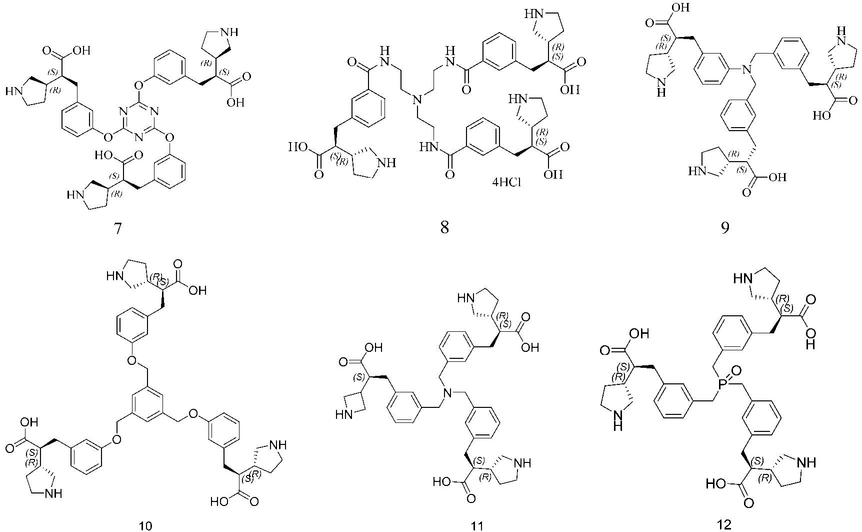 or

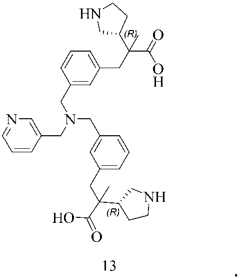

11. A pharmaceutical composition, comprising the compound or the pharmaceutically acceptable salt thereof according to any one of claims 1 to 10.

12. Use of the compound or the pharmaceutically acceptable salt thereof according to any one of claims 1 to 10 for preparing an agent for reducing Lp(a) levels.

13. The use according to claim 12, wherein the compound or the pharmaceutically acceptable salt thereof is used to prepare a drug for treating cardiovascular.
